# EUROPEAN PATENT APPLICATION

(11) **EP 4 516 813 A1**
(43) Date of publication of application: **05.03.2025**
(21) Application number: 23460031.0
(22) Date of filing: 31.08.2023
(51) Int. Cl.: C08B 37/00, C08L 89/00, C08L 89/04

(54) **METHOD FOR PRODUCING COLLAGEN AND GLYCOSAMINOGLYCAN LYOPHILIZATE FROM FISH SKIN**

(30) Priority: 30.08.2023 PL 44594823
(71) Applicant: Uniwersytet Mikolaja Kopernika w Toruniu, 87-100 Torun (PL)
(72) Inventor: Antosik, Pawel, 62-030 Lubon (PL); Burlikowska, Katarzyna, 85-668 Bydgoszcz (PL); Kozlowska, Justyna, 87-100 Torun (PL); Otrocka-Domagala, Iwona, 10-685 Olsztyn (PL); Kaczmarek-Szczepanska, Beata, 87-100 Torun (PL); Zasada, Lidia, 87-807 Wloclawek (PL)

(57) **Abstract**

The method of producing the collagen and glycosaminoglycans lyophilizate from fish skin is characterized in that the skin of the *Salmo salar* species undergoes cleansing, rinsing at least once, and sectioning into pieces, next the skin pieces are placed in an aqueous solution of NaOH at the concentration of 0.01 - 5M and left in the solution for at least 24h, next the skin is removed from the solution and rinsed at least once and placed in acetone, next the skin is removed from the solution and rinsed at least once, and placed in an aqueous solution of acetic acid at the concentration of 0.1 - 5M, next the skin is removed from the solution and in an aqueous solution of NaCl at the concentration of 0.01 - 5M, preferably 0.7M, the solution is then filtered and the undissolved skin fragments are removed and the resulting filtrate is centrifuged and added to a solution of acetic acid at the concentration of 0.1 - 5M, and next dialyzed, frozen, and lyophilized according to standard methods.

## Description

The subject of the invention is a method of producing the collagen and glycosaminoglycans lyophilizate from fish skin.

As a waste product of food processing industry, and due to its biocompatibility, fish skin may constitute the source of compounds which can be applied in the production of dressing materials or implants. It can also be used to coat metal implants, produce wound dressing/hydrogels, and develop scaffolds for cells for the purposes of tissue engineering.

The purpose of the invention is to develop a method of producing compound of collagen and glycosaminoglycans from fish skins in the form of a lyophilizate. The lyophilized form of the product enables its easy storage and distribution in case the invention is launched onto the market. The objective was achieved by the development of an appropriate method and the production of the lyophilizate containing collagen and compounds representing the group of glycosaminoglycans. The resulting lyophilizate shows biocompatible character, and therefore, it can potentially find its biomedical applicability.

The subject of the invention is the method of producing the collagen and glycosaminoglycans lyophilizate from fish skin characterized in that the skin of the *Salmo salar* species undergoes cleansing, rinsing at least once, and sectioning into pieces. Next, the skin pieces are placed in an aqueous solution of NaOH at the concentration of 0.01 - 5M and left in the solution for at least 24h. Then, the skin is removed from the solution and rinsed at least once, and placed in acetone. Further, the skin is removed from the solution, rinsed at least once, and placed in an aqueous solution of acetic acid at the concentration of 0.1 - 5M. In the next step, the skin is removed from the solution and in an aqueous solution of NaCl at the concentration of 0.01 - 5M, preferably 0.7M the solution is then filtered and the undissolved skin fragments are removed and the resulting filtrate is centrifuged and added to a solution of acetic acid at the concentration of 0.1 - 5M, and next dialyzed, frozen, and lyophilized according to standard methods. It is preferable to rinse skin pieces from one to twenty times with water, preferably distilled water. The preferable size for fish skin pieces is from 0.01 cm to 30 cm in maximum diameter or chord. The pieces should preferably be placed in a 0.1M NaOH solution for 1-10 days, preferably 5 days. Preferably the pieces are placed in acetone for 1-10 days, preferably 5 days. Preferably the pieces are placed in an aqueous solution of acetic acid for 1-20 days, preferably 8 days. Preferably the pieces are placed in a solution of NaOH for 1-10 days, preferably 2 days. Preferably the process of dialysis is performed in dialysis tubes in an aqueous solution of 0.1M acetic acid, preferably for 1-10 days. Preferably during dialysis the acid is changed at least once. Preferably the freezing process is carried out at the temperature range from -40 to -5°C, preferably at -18°C, preferably for 1-10 days, most preferably 1 day. Preferably lyophilization is performed for 1-10 days, preferably 2 days.

The method was disclosed in examples of performance which do not exhaust all the possibilities of the invention.

### Example I.

The method of producing the collagen and glycosaminoglycans lyophilizate from fish skin. Skin of the *Salmo salar* species was carefully cleansed by removing remains of attached tissues. Next, it was rinsed with distilled water fivefold and sectioned into small pieces of 1cm² in size with the use of a scalpel.

Stage 1. In order to remove low-collagen proteins, the skin was immersed in a 0.1M NaOH solution for 5 days.

Stage 2. After 5 days, the skin was rinsed with distilled water in order to achieve neutral pH and perform the process of fat removal by immersing in acetone for 1 day.

Stage 3. After 1 day, the skin was carefully rinsed with distilled water and immersed in a 0.5M acetic acid solution for 8 days.

Stage 4. After 8 days, the extracts were filtered out and salted by the addition of NaCl till the final 0.7M extract concentration was reached. The salting process lasted 2 days. The undissolved skin fragments were discarded.

Stage 5. After 2 days the filtrate was centrifuged with the use of the Eppendorf Centrifuge and dissolved in the minimum amount of 0.5M acetic acid . Then, dialysis was conducted with the use of dialysis tubes to 0.1 M acetic acid. 2 days after the dialysis was initiated, the acetic acid solution was exchanged with the fresh portion. The accomplished dialysis process lasted 3 days.

Stage 6. After 3 days the extracts were placed in a freezer (-18°C) for 1 day and then the process of lyophilization was performed with the use of the ALPHA 1-2 LDplus, CHRIST lyophilizer (-20°C, 100Pa) for 2 days in order to obtain dry mass.

Further, the following analyses were carried out for the produced lyophilizate.

### 1. Amino acid analysis

A sample of the lyophilizate was weighed on an analytical digital weight of 0.01 mg accuracy, and the weighed amounts were dissolved in 0.1M acetic acid to the concentration of 30mg protein/1ml. Portions of the solution of 33.3 µl volume, each equivalent to 1mg of the mass of a dry sample, were placed in glass vials for hydrolysis and an amino acid composition analysis was performed according to the procedure described in detail in the Journal of Automatic Chemistry 8 (1986) 170-177. Hydrolysis was conducted in the anaerobic atmosphere, in the gaseous phase, with the use of 6M HCl, with the addition of phenol, for 24h, at the temperature of 110°C. After hydrolysis, the sample was evaporated till dry with a vacuum centrifuge, and the released amino acids were converted to phenylthiocarbamide (PTC) derivatives. The derivatives were dissolved in portions containing 50µl solvent to amino acids, centrifuged for 10 min at 21,000 g, at room temperature, and the volumes of clear supernatants, i.e. 4µl for samples 1 and 2 or 1µl for samples 3 and 4 (which corresponded to 80 or 20µg of the masses of the dry samples respectively) were analyzed with the use of the high-performance liquid chromatography (HPLC) in the PicoTag 3.9x150 mm column (Waters, USA). The quantitative calibration was carried with the use of four different concentrations of factory-made standards of amino acids of Pierce company (USA) enriched with the hydroxyproline standard (Sigma, USA).

### Result:

Table 1 shows the collected results after conversion to nanomoles for particular amino acids in 1 mg of the dry mass of the analyzed sample (mmol/1 mg) as well as after conversion to mole percent of particular amino acids (%mol).

**Table 1. Results of amino acid analysis**

| Amino acid | mmol/1 mg | %mol |
|---|---|---|
| Asx* | 13.10 | 4.46 |
| Glx* | 29.99 | 10.22 |
| HPro | 3.56 | 1.21 |
| Ser | 17.45 | 5.94 |
| Glu | 48.89 | 16.65 |
| His | 8.11 | 2.76 |
| Arg | 15.09 | 5.14 |
| Thr | 14.35 | 4.89 |
| Ala | 28.05 | 9.55 |
| Pro | 19.73 | 6.72 |
| Tyr | 7.46 | 2.54 |
| Val | 16.17 | 5.51 |
| Met | 7.17 | 2.44 |
| Cys-Cys | 1.17 | 0.40 |
| Ile | 11.02 | 3.75 |
| Leu | 19.65 | 6.69 |
| Phe | 8.60 | 2.93 |
| Lys | 24.02 | 8.18 |

| | | |
|---|---|---|
| *Asx=Asp+Asn, Glx=Glu+Gln | | |

### Conclusion:

The presence of amino acids such as glycin (Gly), proline (Pro), and hydroxyproline (HPro) prove the presence of collagen in the analyzed sample since hydroxyproline (HPro) can hardly be found in nature excluding collagen.

### 2. Sugars determination analysis

Sugars were determined with the HPLC method using the Rezex RHM Monosaccharide H+, 7.8 x 300 mm column, Phenomenex; the temperature of the column: 60°C; the flow rate: 0.6 ml/min; detection: R; Program of analysis: isocratic; eluent: H₂0.

The resulting lyophilizate of hydrolates was dissolved in the amount of water sufficient to dissolve it and controlled to keep the concentration at the possibly highest level. The solution was filtered with the use of sample preparation filters.

### Results:

As a result of the hydrolysis of sugars glucose was identified (retention time: 9.9-10.09 min), and the peak area was equal to 1.059% for the amount of 4.59 mg/ml of the lyophilizate.

### 3. Cytocompatibility test (MTT)

The method was based on the ISO 10993-5 guidelines concerning the biocompatibility assessment of medical device as well as the cytotoxicity agar diffusion approved in the standard. The methodology allows the qualitative assessment of the ability to induce the toxicity effect by a medical device. After initial cultivation on plates, mice fibroblast cells are poured over with a medium mixed with agar of the final 1% concentration. After 24h, samples of the tested product as well as control samples are deposited on the solidified agar layer, and they are all incubated for 24 +/- 2 h at the temperature of 37+/- 1°C. After 24h incubation, the cell culture is stained with a solution of MTT in order to assess dead cells. The assessment is performed by measuring the cell growth inhibition zones observed as those lacking the absorbed stain.

According to the ISO 10993-5 standard, if the toxicity level assessment is higher than 2, the product/device is classified as that producing the cytotoxic effect.

### Result: 0

Conclusion : The lyophilizate does not induce the cytotoxic effect.

### 4. In vivo test

### Lyophilizate implantation

The experiment was conducted on 24 male rats Wistar Cmdb:WI aged above 9 weeks (adult males) (born on 20-30 Sep. 2022).

Stage 1. Dealing with animals from the control group (n=12):
The inhalation method with isoflurane was applied for general anesthesia. Each animal was placed singly in an induction chamber (5% isoflurane, 1.2 l/min flow rate) till its postural reflexes were lost. After removal from the chamber, the animals were put on heating plates to avoid hypothermia. The anesthetic effect was maintained with the use of anesthesia masks (3% isoflurane, 0.5 l/min flow rate). In the next step, animal hair was removed with an electric shaver around the shoulder blade (0.5cm x 1cm area). The thus prepared surgery area was disinfected (iodine tincture). Next, the skin was sectioned, ca 0.3 cm, and the wound was stitched (4-0 resorbable thread). When the procedure was accomplished, the animals were woken up and transferred to their living area.

Dealing with animals from the experimental group (n=12):
The inhalation method with isoflurane was applied for general anesthesia. Each animal was placed singly in an induction chamber (5% isoflurane, 1.2 l/min flow rate) till its postural reflexes were lost. After removal from the chamber, the animals were put on heating plates to avoid hypothermia. The anesthetic effect was maintained with the use of anesthesia masks (3% isoflurane, 0.5 l/min flow rate). In the next step, animal hair was removed with an electric shaver around the shoulder blade (0.5cm x 1 cm area). The thus prepared surgery area was disinfected (iodine tincture). Next, the skin was sectioned, ca 0.3 cm, an implant was introduced (0.5 g, in the solid state) and the wound was stitched (4-0 resorbable thread). When the procedure was accomplished, the animals were woken up and transferred to their living area.

Stage 2. Within the next 6 days, the animals condition and behavior were monitored every day. No health issue or fall was observed.

Stage 3. On the 7th day after implantation, 4 animals from each group, i.e. experimental and control, were euthanized. Sedation was injected intramuscularly using medetomidine (Domitor) in the amount of 0.5mg/kg of body mass, and then Euthosal was introduced intraperitoneally (140mg/ kg). Posthumously, fragments of subcutaneous tissue (ca 2cm x 2cm) (with implants in the experimental group), were collected to carry out histological tests. The condition and behavior of the remaining animals were monitored. No health issue or fall was observed.

Stage 4. On the 14th day after the lyophilizate implantation, 4 animals from each group, i.e. experimental and control, were euthanized. The whole procedure was repeated in the same manner as on the 7th day after implantation. The condition and behavior of the remaining animals were monitored. No health issue or fall was observed.

Stage 5. On the 21st day after the lyophilizate implantation, 4 animals from each group, i.e. experimental and control, were euthanized. The whole procedure was repeated in the same manner as on the 7th and 14th day after implantation. Posthumously, no differences in the macroscopic appearance of the stitching areas were found. The wounds were healed and the stitching areas were covered with hair in both groups.

### Histological examination

Tissue samples were immediately fixed in 10% buffered formalin and were routinely processed for the purposes of histopatological examination with the use of the paraffin method, sectioned at 5 m and stained with hematoxylin and eosin according to Mayer method. The blind assessment of the samples was performed by an experienced pathologist. Microimages were taken using the Olympus BX43 microscope equipped with the Olympus SC 180 camera (Hamburg, Germany) as well as the cellSens software (Olympus).

### Results

### The control group:

- 7 days after implantation:
   The epidermis over the lesion shows moderately irregular proliferation, parakeratotic hyperkeratosis, with the formation of a rather extensive intracorneal pustule filled with neutrophils, protein fluid, and erythrocytes. The lesion surface is focally ulcerated. In the dermis, hyperplasia of moderately mature granulation tissue is present, fragments of surgical thread are covered with a microphage infiltrate, less numerous neutrophiles, scarce eosinophiles.
   Diagnosis: granulation and resorption of surgical stitches
- 14 days after implantation:
   The presence of the prominence of skin covered with epidermis showing significant degree of irregular proliferation, moderate ortho- and parakeratotic hyperkeratosis was observed. A small intracorneal pustule filled with erythrocytes, protein fluid, and neutrophils is formed. In the dermis, there is rather mature granulation tissue, fragments of surgical thread, surrounded by a moderate infiltration of macrophages, multinucleated giant cells, lymphocytes, plasma cells, and a few neutrophils.
   Diagnosis: granulation and resorption of surgical stitches
- 21 days after implantation:
   The presence of the prominence of skin covered with epidermis showing moderate irregular proliferation and orthokeratotic hyperkeratosis was observed. In the dermis, rather mature granulation tissue, fragments of surgical thread are present. Within granulation, scarce lymphocyte, plasmocyte, and microphage infiltrates are observed.
   Diagnosis: granulation and resorption of surgical stitches

### The examined group:

- 7 days after implantation:
   The presence of the prominence of skin covered with epidermis showing considerable and irregular proliferation, ortho- and parakeratotic hyperkeratosis, with the formation of intracorneal pustules filled with erythrocytes, protein fluid, and neutrophils was observed. In the dermis, moderately mature granulation tissue and surgical thread covered with a moderate lymphocyte, plasmocyte, and macrophage infiltrate are present; the threads sometimes calcify. In the hypodermis, hyperplasia of moderately mature granulation tissue is observed; within granulation tissue, exogenous material incrusted with calcium salts, surrounded by a small to moderate size microphage, lymphocyte, and single eosinophil infiltrate is present.
   Diagnosis: granulation and resorption of surgical stitches and their calcification, the implant undergoes resorption with granulation and minor inflammation
- 14 days after implantation:
   The presence of the prominence of skin covered with epidermis showing moderate and irregular proliferation, ortho- and parakeratotic hyperkeratosis, with the formation of intracorneal pustules filled with erythrocytes, protein fluid, and neutrophils was observed. An intracorneal pustule filled with erythrocytes, protein fluid, and neutrophils is formed. In the dermis, rather mature granulation tissue, small lymphocyte and plasmocyte infiltrates are present. In the hypodermis, hyperplasia of moderately mature granulation tissue is observed; within granulation tissue, fragments of exogenous material of various sizes, incrusted with calcium salts, covered with a moderate microphage, multinucleated giant cell, lymphocyte and plasmocyte infiltrate are present.
   Diagnosis: granulation with minor inflammation, the implant undergoes resorption with granulation and minor inflammation
- 21 days after implantation:
   The presence of the prominence of skin covered with epidermis showing moderate and irregular proliferation as well as orthokeratotic hyperkeratosis is observed. In the dermis, mature granulation tissue with small lymphocyte, plasmocyte, and macrophage infiltrates as well as surgical stitches incrusted with calcium salts are present. In the hypodermis, hyperplasia of rather mature granulation tissue is observed; within granulation tissue, small fragments of exogenous material incrusted with calcium salts, surrounded by a moderate microphage, few multinucleated giant cell, lymphocyte and plasmocyte infiltrate are present.

### Example II.

The method of producing the collagen and glycosaminoglycans lyophilizate from fish skin is that the skin of the *Salmo salar* species is cleansed, rinsed once and sectioned into pieces. Next, skin sections are placed in an aqueous solution of NaOH at the concentration of 0.01M and left in solution for 24h. Next, skins are removed from the solution, rinsed once, and placed in acetone. Next, they are removed from the solution, rinsed once, and placed in an aqueous solution of acetic acid at the concentration of 0.1M. Next, skin pieces are removed from the solution and in aqueous solution of NaCl at the concentration of 0.01M the solution is then filtered and the undissolved skin fragments are removed. The resulting filtrate is centrifuged and added to a solution of acetic acid at the concentration of 0.1M and next dialyzed, frozen, and lyophilized according to standard methods. The fish skin pieces are of 0.01 cm. The skin pieces are placed in a 0.1M NaOH solution as well as in acetone for 1 day. Skin pieces are placed in an aqueous solution of acetic acid for 1 day. The process of dialysis is performed in dialysis tubes in an aqueous solution of 0.1M acetic acid for 1 day. Freezing is carried out at the temperature of -40°C for 1 day. Lyophilization is performed for 1 day.

### Example III.

The method of producing the collagen and glycosaminoglycans lyophilizate from fish skin is that the skin of the *Salmo salar* species is cleansed, rinsed 20 times, and sectioned into pieces. Next, skin pieces are placed in an aqueous solution of NaOH at the concentration of 5M and left in solution for 240h. Next, the skins are removed from the solution, rinsed tenfold, and placed in acetone. Next skins are removed from the solution, rinsed fivefold, and placed in an aqueous solution of acetic acid at the concentration of 5M. Next, skins are removed from the solution and in an aqueous solution of NaCl at the concentration of 5M the solution is then filtered and the undissolved skin fragments are removed. The resulting filtrate is centrifuged and added to a solution of acetic acid at the concentration of 5M and next dialyzed, frozen, and lyophilized according to standard methods. Each time rinsing is carried out with distilled water. The fish skin pieces are of 30 cm. The skin pieces are placed in the solution 0.1M NaOH as well as in acetone for 10 days. The skin pieces are placed in an aqueous solution of acetic acid for 20 days. The process of dialysis is performed in dialysis tubes in an aqueous solution of 0.1M acetic acid for 10 days. During dialysis, the acid was completely changed at twice. Freezing is carried out at the temperature of -5°C for 10 days. Lyophilization is performed for 10 days.

## Claims

1. The method of producing the collagen and glycosaminoglycans lyophilizate from fish skin is **characterized in that** the skin of the *Salmo salar* species undergoes cleansing, rinsing at least once, and sectioning into pieces, next, the pieces of skin are placed in an aqueous solution of NaOH at the concentration of 0.01 - 5M and left in the solution for at least 24 h, next, the skin is removed from the solution, rinsed at least once, and placed in acetone, next, the skin is removed from the solution, rinsed at least once, and placed in an aqueous solution of acetic acid at the concentration of 0.1 - 5M, next, the skin is removed from the solution and in an aqueous solution of NaCl at the concentration of 0.01 - 5M, preferably 0.7M the solution is then filtered and the undissolved skin fragments are removed and the resulting filtrate is centrifuged and added to a solution of acetic acid at the concentration of 0.1 - 5M, and next dialyzed, frozen, and lyophilized according to standard methods.

2. Method according to claim 1 **characterized in that** pieces are rinsed one to twenty times and rinsing is performed with water, preferably distilled.

3. Method according to claim 1 or 2 **characterized in that** fish skin pieces are of 0.01 cm to 30 cm in the longest diameter or chord.

4. Method according to claim 1,2 or 3 **characterized in that** skin pieces are placed in a 0.1M NaOH solution for 1 to 10 days, preferably 5 days.

5. Method according to claim 1,2,3 or 4 **characterized in that** skin pieces are placed in acetone for 1 to 10 days, preferably 5 days.

6. Method according to claim 1,2,3,4 or 5 **characterized in that** skin pieces are placed in an aqueous solution of acetic acid for 1 to 20 days, preferably 8 days.

7. Method according to claim 1,2,3,4,5 or 6 **characterized in that** skin pieces are placed in a solution of NaOH for 1 to 10 days, preferably 2 days.

8. Method according to claim 1,2,3,4,5,6 or 7 **characterized in that** the process of dialysis is performed in dialysis tubes in an aqueous solution of 0.1M acetic acid, preferably for 1 to 10 days.

9. Method according to claim 1,2,3,4,5,6,7 or 8 **characterized in that** during dialysis the acid is changed at least once.

10. Method according to claim 1,2,3,4,5,6,7,8 or 9 **characterized in that** the process of freezing is carried out at the temperature between -40°C to -5°C, preferably -18°C, preferably for 1 to 10 days, most preferably for 1 day.

11. Method according to claim 1,2,3,4,5,6,7,8,9 or 10 **characterized in that** the process of lyophilization is carried out for 1 to 10 days, preferably 2 days.
